(19) Europäisches Patentamt
European Patent Office
Office européen des brevets

(11) Publication number: **0 207 651 A2**

# (12) EUROPEAN PATENT APPLICATION

(21) Application number: **86304353.5**

(22) Date of filing: **06.06.86**

(51) Int. Cl.⁴: **C07D 487/04**, A61K 31/53, //(C07D487/04,251:00,231:00)

(30) Priority: **06.06.85 US 741819**

(43) Date of publication of application: **07.01.87 Bulletin 87/02**

(84) Designated Contracting States: **AT BE CH DE FR GB IT LI LU NL SE**

(71) Applicant: **BIOMEASURE INC.**
**11-15 East Avenue Hopkinton Industrial Park**
**Hopkinton Massachusetts 01748(US)**

(72) Inventor: **Kim, Sun Hyuk**
**20 Whitney Street**
**Chestnut Hill Massachusetts 02167(US)**

(74) Representative: **Sheard, Andrew Gregory**
**Kilburn & Strode 30, John Street**
**London WC1N 2DD(GB)**

## (54) Anti-inflammatory 7-phenyl pyrazolo-[1,5A]-1,3,5-triazine derivatives.

(57) Compounds of general formula 11:

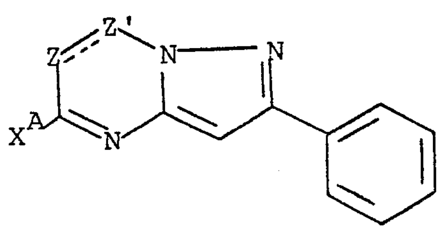

wherein either $X^A$ represents a thioalkyl group and the $-Z{=}{=}{=}Z'-$ moiety represents $-N=\overset{Y}{\overset{|}{C}}-$ , wherein Y represents a hydroxyalkylamino, dialkylamino, carboxyalkylamino, N-heterocycloalkyl, heteroalkylthioalkylamino, alkylthiocarboxyalkylamino or heteroarylthioalkylamino group,

or $X^A$ represents a thioalkyl thioaralkyl or thioheteroaralkyl group and the $-Z{=}{=}{=}Z'-$ moiety represents $-\underset{}{\overset{B}{\overset{|}{N}}}-\overset{O}{\overset{\|}{C}}-$ , wherein B represents a hydrogen atom or a carboalkoxy, alkyl, allyl or benzyl group, and their salts, are non-steroidal anti-inflammatory agents, such as are useful in the treatment of arthritis.

EP 0 207 651 A2

## ANTI-INFLAMMATORY 7-PHENYL PYRAZOLO-[1,5-A]-1,3,5,-TRIAZINE DERIVATIVES

This invention relates to non-steroidal anti-inflammatory agents, such as are useful for the treatment of arthritis.

In its broadest aspect, the present invention provides a compound of general formula (11)

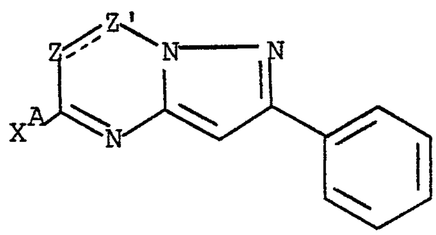

(11)

wherein either $X^A$ represents a thioalkyl group and the -Z===Z'- moiety represents 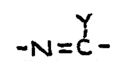, wherein Y represents a hydroxyalkylamino, dialkylamino, carboxyalkylamino, N-heterocycloalkyl, heteroalkylthioalkylamino, alkylthiocarboxyalkylamino or heteroarylalkylthioalkylamino group,

or $X^A$ represents a thioalkyl thioaralkyl or thioheteroaralkyl group and the -Z===Z'- represents 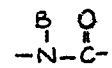, wherein B represents a hydrogen atom or a carboalkoxy, alkyl, allyl or benzyl group,

or a pharmaceutically or veterinarily acceptable salt thereof.

The invention encompasses compounds having anti-arthritic activity and being of general formula 1:

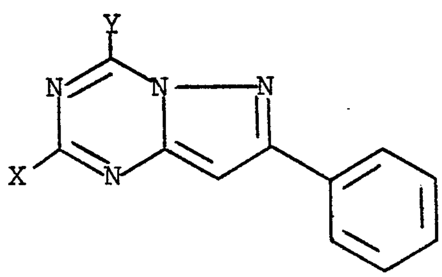

(1)

wherein X represents a thioalkyl (e.g. thiomethyl) group; and Y represents a dialkylamino (e.g. dimethylamino), hydroxyalkylamino (e.g. ethanolamino), carboxy-alkylamino, (e.g. carboxyethylamino) N-heterocycloalkyl (e.g. thiazolidinyl), heteroalkylthioalkylamino (e.g. ethylthioethylamino), alkylthiocarboxyalkylamino or heteroarylalkylthioalkylamino (e.g. 2-pyridyl-methylthioethylamino) group. Preferably, all alkyl and allyl groups contain between 1 and 4 carbon atoms, inclusive, and all aryl groups contain a single phenyl ring.

Preferred embodiments include:

the compound of general formula 1, wherein X is $-SCH_3$; and Y is $-NHCH_2CO_2H$ (i.e. 4-carboxymethylamino-2-methylthio-7-phenylpyrazolo [1,5-a]-1,3,5-triazine); the compound of general formula 1, wherein X is $-SCH_3$; and Y is

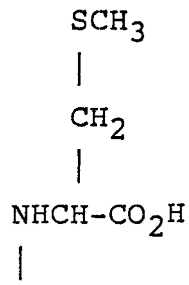

(i.e. 4-[(2-methylthio-1-carboxy)]ethylamino-2-methylthio-7-phenylpyrazolo [1,5-a]-1,3,5-triazine);

the compound of general formula 1, wherein X is -$SCH_3$; and Y is

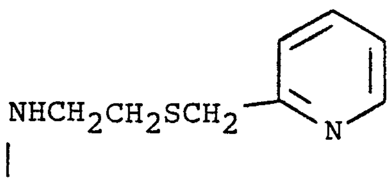

(i.e. 2-methylthio-7-phenyl-4-[2-(2-(pyridyl) methylthio] ethylamino-pyrazolo [1,5-a]-1,3,5-triazine);

the compound of general formula 1, wherein X is -$SCH_3$; and Y is -$NHCH_2CH_2OH$;

(i.e. 4-(2-ethanol)amino-2-methylthio-7-phenyl-pyrazolo [1,5-a]-1,3,5-triazine);

the compound of genral formula 1, wherein X is -$SCH_3$; and Y is $N(CH_3)_2$;

(i.e. 4-dimethylamino-2-methylthio-7-phenyl-pyrazolo[1,5-a]-1,3,5-triazine);

the compound of general formula 1, wherein X is -$SCH_3$; and Y is

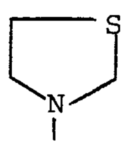

(i.e. 4-thiazolidinyl-2-methylthio-7-phenylpyrazolo -[1,5-a]-1,3,5-triazine);

and pharmaceutically and veterinarily acceptable salts of any of these compounds.

The invention also encompasses compounds of general formula 2:

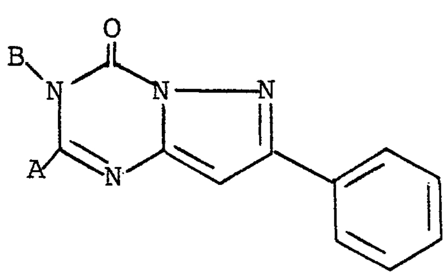

(2)

wherein A represents a thioalkyl, thioaralkyl (e.g. thiobenzyl), or thioheteroaralkyl (e.g. 2, 3, or 4-picolyl) group; and B represents a hydrogen atom, or a carboalkoxy, alkyl, allyl, or benzyl group. Preferably, all allylic and alkyl groups contain between 1 and 4 carbon atoms, inclusive, and all aryl groups contain a single (e.g. phenyl) ring.

Preferred embodiments include the compound of general formula 2, wherein A is $-SCH_3$; and B is

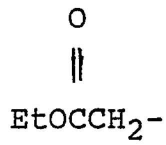

(i.e. 3-carbethoxymethyl-2-methylthio-7-phenyl-pyrazolo [1,5-a]-1,3,5-triazine-4-one);

the compound of general formula 2, wherein A is

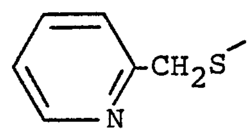

and B is H (i.e. 2-(2-pyridylmethyl) thio-7-phenyl-pyrazolo [1,5-a]-1,3,5-triazine-4-one);

the compound of general formula 2, wherein A is

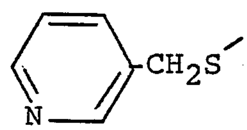

and B is H (i.e. 2-(3-pyridylmethyl) thio-7-phenyl-pyrazolo [1,5-a]-1,3,5-triazine-4-one);

the compound of general formula 2, wherein A is

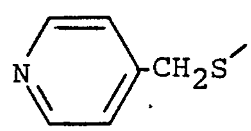

and B is H (i.e. 2-(4-pyridylmethyl) thio-7-phenyl-pyrazolo [1,5-a]-1,3,5-triazine-4-one);

the compound of general formula 2, wherein A is

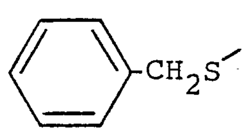

and B is H (i.e. 2-benzylthio-7-phenylpyrazolo [1,5-a]-1,3,5-triazine-4-one);

the compound of general formula 2, wherein A is -$SCH_3$; and B is

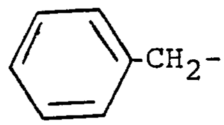

(i.e. 3-benzyl-2-methylthio-7-phenylpyrazolo [1,5-a]-1,3,5-triazine-4-one);

or a pharmaceutically or veterinarily acceptable salt of any of these compounds.

The invention in other aspects provides: a compound as described above for use in medicine; a pharmaceutical or veterinary composition comprising a compound as described above and a pharmaceutically or veterinarily acceptable carrier therefor; and the use of a compound as described above in the preparation of an anti-inflammatory agent.

In addition to anti-arthritic activity, when a therapeutically effective amount of the compound is administered in a pharmaceutically acceptable carrier, e.g. magnesium crbonate or lactose, the compounds have antiulcer activity against ulcers induced by dimaprit and indomethacin, and can provide anti-inflammatory action without gastric irritation.

When injected or administered in the form of a pill, tablet, capsule, or liquid, the compounds are non-toxic, non-mutagenic, stable and, in the case of the orally administerable formulations (which are preferred), will pass through the stomach without losing their effectiveness.

Other features and advantages will be apparent from the following description of the preferred embodiments, and from the claims.

Description of the Preferred Embodiments

Structure

The compounds of the invention have the general formula (11), which may be classified into general formulae (1) and (2). Examples of preferred compounds are those referred to as preferred embodiments above.

The compounds are derivatives of 7-phenylpyrazolo [1,5-a]-1,3,5-triazine having a nitrogen at the ring junction. All the compounds can exhibit tautomerism, and the formulae are intended to cover all tautomers.

The compounds or pharmaceutically acceptable salts therefor can be administered alone or in combination with a pharmaceutically acceptable carrier or diluent.

Acceptable salts include hydrochlorides, hydrobromides, and sulphates. Particularly useful organic acid salts are citrates, acetates, maleates, and fumarates.

For oral administration the pharmaceutical composition can most conveniently be in the form of capsules or tablets, which may be slow release tablets. The composition can also be in the form of a dragee or syrup.

Synthesis

A process for the preparation of compounds of general formula (11) also forms part of the invention and comprises either

(i) reacting an amine of the formula Y-H, where Y is as defined for general formula 11 above with a compound of general formula 8:

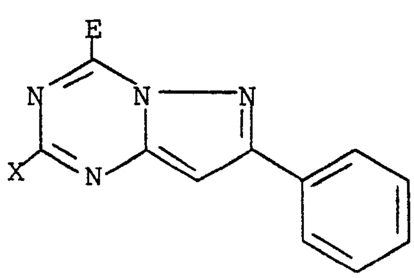

(8)

wherein E represents a leaving group and X represents a thioalkyl group; or

(ii) condensing a compound of general formula 9:

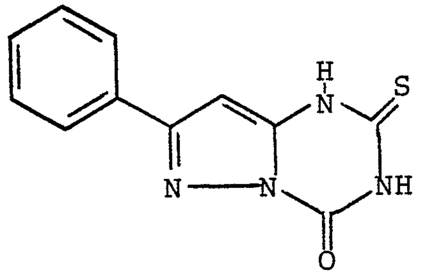

(9)

with a condensing agent which alkylates, aralkylates or thioheteroaralkylates at the sulphur atom and optionally substituting the nitrogen atom which is between and next to the carbon atoms bearing the oxygen and sulphur atoms with a carboalkoxy, alkyl, allyl or benzyl group, or

(iii) converting a compound of formula 11 so formed into a further compound of formula 11;

(iv) and optionally, in any of the above cases, forming a pharmaceutically or veterinarily acceptable salt of the compound.

More specifically, compounds of formula (1) may be synthesized as follows.

The starting material is an amine of formula - (3), (4), (5), (6) or (7):

(3) $R^2$ $R^3$ >NH where $R^2$ and $R^3$ are the same or different lower ($C_1$-$C_4$) alkyl groups;

(4) $HO(CH_2)_n NH_2$

wherein n is equal to 2-6, inclusive;

(5)

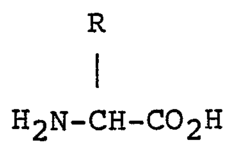

wherein R is H, or the identifying group of the L or D form of an amino acid;

(6)

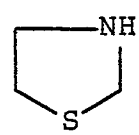

(7)

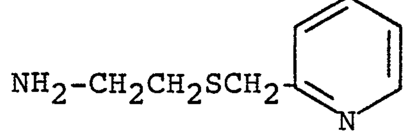

The amine undergoes a condensation reaction with a reagent such as

(8)

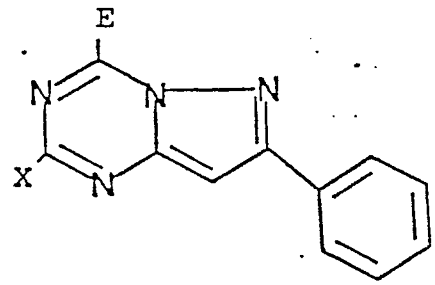

where E is a good leaving group, e.g. a halogen - (e.g. Cl or Br), or alkoxy (e.g. methoxy or ethoxy). X in formula 8 is as defined above for formula (1).

The condensation reaction is preferably carried out in an inert protic solvent, e.g. water, alcohol, ethoxyethanol, tetrahydrofuran, acetonitrile, dimethylformamide, or a mixture of these solvents at room temperature. If necessary, a base such as KOH, NaOH, $K_2CO_3$, $NaHCO_3$, or triethylamine can be used as an acid scavenger.

Compounds within formula (8) can be synthesized according to known methods, e.g. Capuno et al, Chem. Ber. 104, 3039 (1974); J. Kobe et al, J. Het. Chem., 11, 199 (1974); K. Senga et al, J. Het. Chem., 12, 893 (1973) and J. Med. Chem., 25, 243 (1982). The amine compounds within formulae (3), (4), (5), (6) and (7) are commercially available or can be synthesized according to standard methods, e.g. G. J. Durant et al , J. Med. Chem., 20, 901 (1977).

The compounds of Formula (2) can be synthesized as follows. A compound of Formula (9)

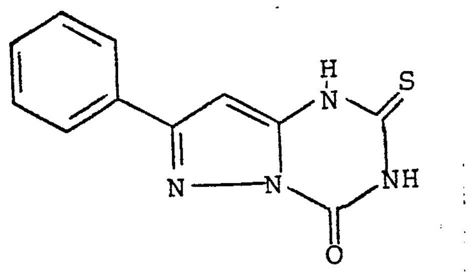

(9)

is condensed with an alkylating agent, e.g. alkyl halide (e.g. methyl iodide), dialkyl sulphate (e.g. dimethylsulphate), aralkyl halide (e.g. benzyl chloride), or heteroaralkyl halide (e.g. 2, 3 or 4-(picolyl) chloride hydrochloride). If desired, the initial condensation product is further substituted with an allyl halide (e.g. allyl chloride), aralkyl halide (e.g. benzyl chloride), or

(10) $Z\text{-}CH_2\text{-}COOR'$

wherein Z represents a halogen atom and R' represents an alkyl group (e.g. methyl). All condensation reactions are preferably carried out in the presence of base (e.g. NaOH, $Na_2CO_3$) in an inert protic solvent described previously.

The above-described alkylating agents are commercially available or can be synthesized according to known methods, e.g. K. Senga et al, J. Het. Chem., 12 893 (1975).

The following examples illustrate but do not limit the invention.

Example 1

4-[(3-methylthio-1-carboxy)]propylamino-2-methylthio-7-phenylpyrazolo [1,5-a]-1,3,5-triazine

1.2ml 1N-NaOMe is added to a suspension of L-methionine (150 mg) in 5 ml methanol, followed by 290 mg of 4-methoxy-2-methylthio-7-phenylpyrazolo [1,5-a]-1, 3, 5-triazine and the mixture is stirred at room temperature for 1 hr. After evaporation of solvent in vacuo, the residue is partitioned between water and $CHCl_3$. The aqueous layer is acidified with 1N-HCl to precipitate 300 mg of a pale yellow solid. The solid is collected by filtration, washed with water, and then dried. m.p. 225°C - (decomposes). TLC (silica gel: $CHCl_3/MeOH = 3:1$)-$R_f = 0.39$. Anal. cal'cd for $C_{17}H_{19}N_5O_2S_2$: C, 52.41; H, 4.91; N, 17.98. Found: C, 51.97; H, 4.80; N, 17.91.

Example 2

2-methylthio-7-phenyl-4-[2-[(2-pyridyl) methylthio]-ethylamino]-pyrazolo [1,5-a]-1,3,5-triazine

4 ml 1N-NaOMe is added to a suspension of 270 mg of 4-methoxy-2-methylthio-7-phenyl-pyrazolo [1,5-a]-1,3,5-triazine and 520 mg of 2-pyridylmethylthioethyl-amine dihydrobromide in 10 ml MeOH. After stirring at room temperature for 1 hr., the solvent is evaporated in vacuo, and the residue is partitioned between water and chloroform. The chloroform layer is washed with water several times, then dried over $MgSO_4$. After the removal of solvent, the residue is purified by chromatographing on silica gel (30 g) using chloroform, followed by chloroform-acetone (19:1) as eluants. Appropriate fractions are collected, and the solvent removed to give 280 mg of a pale yellow solid. m.p. 119-120°C. TLC (silica gel: $CHCl_3$/acetone = 9:1)$R_f$ = 0.42. Anal. cal'cd. for $C_{20}H_{20}N_6S_2$ (0.4 $H_2O$): C, 58.15; H, 5.00; N, 20.34. Found: C, 57.97, H. 4.89; N, 20.03.

Examples 3 to 6

4-carboxymethylamino-2-methylthio-7-phenylpyrazolo [1-5-a]-1,3,5-triazine; 4-(2-ethanol)-amino-2-methylthio-7-phenylpyrazolo[1,5-a]-1,3,5-triazine; 4-thiazolidinyl-2-methylthio-7-phenyl-pyrazolo [1,5-a]-1,3,5-triazine and 4-dimethylamino-2-methylthio-7-phenylpyrazolo [1,5-a]-1,3,5-triazine are prepared in analogous fashion by making appropriate modifications of the procedures described in Examples 1 and 2.

Example 7

2-(2-pyridylmethyl)thio-7-phenylpyrazolo [1,5-a]-1,3,5-triazine-4-one

3 ml 2N-NaOH is added to a suspension of 490 mg of 7-phenyl-2-thiopyrazolo [1,5-a]-1,3,5-triazine-4-one in 10 ml ethanol. After a clear solution is obtained, a solution of 330 mg of 2-picolyl chloride hydrochloride in 2 ml ethanol is added and the mixture is stirred at room temperature for 1 hr. The mixture is acidified with acetic acid (pH 4) and the resulting colourless precipitate is collected by filtration. The precipitate is washed sequentially with water, ethanol, and ether, then dried to yield 590 mg of colourless solid. m.p. 233-234°C - (decomposition). TLC (silica gel: $CHCl_3$/MeOH = 9:1)$R_f$ = 0.47. Anal. cal'cd for $C_{17}H_{13}N_5OS$: C, 60.87; H, 3.90; N, 20.88. Found: C, 61.02; H, 3.86; N, 20.89.

Example 8

3-Carbethoxymethyl-2-methylthio-7-phenylpyrazolo [1,5-a]-1,3,5-triazine-4-one

A mixture of 260 mg of 2-methylthio-7-phenyl-pyrazolo [1,5-a]-1,3,5-triazine-4-one and 140 mg of anhydrous potassium carbonate in 5 ml dry dimethylformamide is stirred at room temperature for 15 min while 0.14 ml ethylbromacetate is added dropwise. The mixture is stirred at room temperature overnight, filtered, and the filter cake washed with dimethylformamide.

After evaporation fo solvent in vacuo, the residue is partitioned between water and chloroform. The chloroform layer is washed with water, then dried over $MgSO_4$. The solvent is evaporated in vacuo to dryness. Recrystallization of the residue from ethanol yields 170 mg of needle-like crystals with a melting range of 173-174°C. Mass spectral analysis shows a molecular ion of M/e 344.

Examples 9 to 12

2-(3-pyridylmethyl)thio-7-phenylpyrazolo [1,5-a]-1,3,5-triazine-4-one; 2-(4-pyridylmethyl)thio-7-phenylpyrazolo [1,5-a]-1,3,5-triazine-4-one; 2-benzylthio-7-phenylpyrazolo [1,5-a]-1,3,5-triazine-4-one; and 3-benzyl-2-methylthio-7-phenylpyrazolo - [1,5-a]-1,3,5-triazine-4-one are prepared in analogous fashion by making appropriate modifications of the procedures described in Examples 7 and 8.

Pharmacology

When administered to mammals alone or together with a pharmaceutically acceptable carrier substance (e.g. orally, topically, intravenously, parenterally, nasally, or by suppository), the compounds of the invention are useful for the treatment of arthritis. The compounds of the invention can also be used to prevent peptic ulcers, and to treat reflux esophagitis, acute erosive gastritis, and pancreatic insufficiency.

The compounds of the invention inhibit ulcers induced by non-steroidal anti-inflammatory drugs, e.g. aspirin and indomethacin, without inhibiting their anti-inflammatory and analgesic activity. Thus the compounds can be particularly useful in treating or preventing gastric ulcers in patients, e.g. arthritics, who consume non-steroidal anti-inflammatory drugs. The anti-inflammatory action of the compounds can even reduce or eliminate the dosage required of non-steroidal anti-inflammatory drugs.

The compounds can be adminstered to a mammal in a dosage of 5 to 100 mg/kg/day, preferably 10 to 50 mg/kg/day.

Other embodiments are within the following claims.

**Claims**

1. A compound of general formula (11)

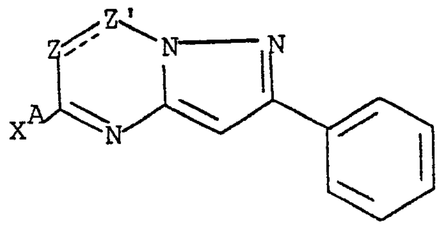

(11)

wherein either $X^A$ represents a thioalkyl group and the 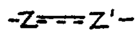 moiety represents 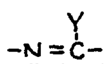, wherein Y represents a hydroxyalkylamino, dialkylamino, carboxyalkylamino, N-heterocycloalkyl, heteroalkylthioalkylamino, alkylthiocarboxyalkylamino or heteroarylalkylthioalkylamino group,

or $X^A$ represents a thioalkyl thioaralkyl or thioheteroaralkyl group and the 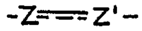 moiety represents 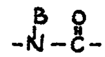, wherein B represents a hydrogen atom or a carboalkoxy, alkyl, allyl or benzyl group,

or a pharmaceutically or veterinarily acceptable salt thereof.

2. A compound as claimed in Claim 1 and of general formula 1:

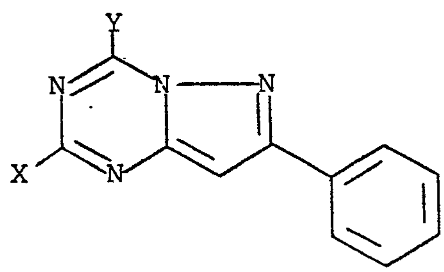

(1)

wherein X represents a thioalkyl group and Y represents a hydroxyalkylamino, dialkylamino, carboxyalkylamino, N-heterocycloalkyl, heteroalkylthioalkylamino, alkylthiocarboxyalkylamino, or heteroarylalkylthioalkylamino group;

or a pharmaceutically or veterinarily acceptable salt thereof.

3. A compound as claimed in Claim 1 and of general formula 2:

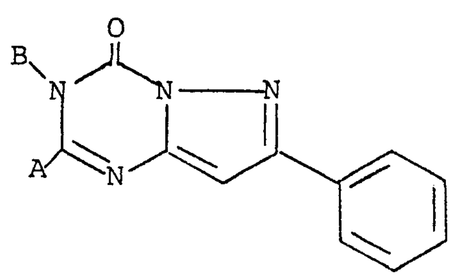

(2)

wherein A represents thioalkyl, thioaralkyl, or thioheteroaralkyl group; and B represents a hydrogen atom or a carboalkoxy, alkyl, allyl or benzyl group;

or a pharmaceutically or veterinarily acceptable salt thereof.

4. 4-Carboxymethylamino-2-methylthio-7-phenylpyrazolo [1,5-a]-1,3,5-triazine; 4-[(3-methylthio-1-carboxy)] propylamino-2-methylthio-7-phenylpyrazolo [1,5-a]-1,3,5-triazine; 2-methylthio-7-phenyl-4-[2-(2-pyridyl) methylthio]ethylamino]-pyrazolo [1,5-a]-1,3,5-triazine; 4-dimethylamino-2-methylthio-7-phenylpyrazolo [1,5-a]-1,3,5-triazine; 4-(2-ethanol)amino-2-methylthio-7-phenylpyrazolo [1,5-a]-1,3,5-triazine; 4-thiazolidinyl-2-methylthio-7-phenylpyrazolo [1,5-a]-1,3,5-triazine or a pharmaceutically or veterinarily acceptable salt of any of these compounds.

5. 3-Benzyl-2-methylthio-7-phenylpyrazolo [1,5-a]-1,3,5-triazine-4-one; 3-carbethoxymethyl-2-methylthio-7-phenylpyrazolo [1,5-a]-1,3,5-triazine-4-one; 2-(2-pyridylmethyl)thio-7-phenylpyrazolo [1,5-a]-1,3,5-triazine-4-one; 2-(3-pyridylmethyl)thio-7-phenylpyrazolo [1,5-a]-1,3,5-triazine-4-one; 2-(4-pyridylmethyl)thio-7-phenylpyrazolo [1,5-a]-1,3,5-triazine-4-one; 2-benzylthio-7-phenylpyrazolo [1,5-a]-1,3,5-triazine-4-one; or a pharmaceutically or veterinarily acceptable salt of any of these compounds.

6. A compound as defined in any one of Claims 1 to 6 for use in human or veterinary medicine.

7. A pharmaceutical or veterinary composition comprising a compound as defined in any one of Claims 1 to 6 and a pharmaceutically or veterinarily acceptable carrier therefor.

8. The use of a compound as defined in any one of Claims 1 to 6 in the preparation of an anti-inflammatory agent.

9. A process for the preparation of a compound of general formula 11:

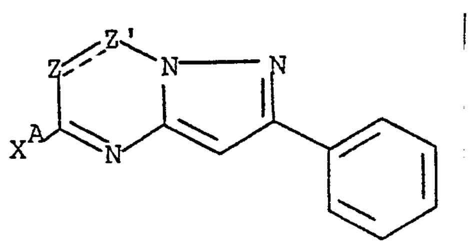

(11)

wherein either $X^A$ represents a thioalkyl group and the -Z=Z'- moiety represents 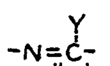, wherein Y represents a hydroxyalkylamino, dialkylamino, carboxyalkylamino, N-heterocycloalkyl, heteroalkylthioalkylamino, alkylthiocarboxyalkylamino or heteroarylalkylthioalkylamino group,

or $X^A$ represents a thioalkyl thioaralkyl or thioheteroaralkyl group and the -Z=Z'- moiety represents 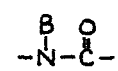, wherein B represents a hydrogen atom or a carboalkoxy, alkyl, allyl or benzyl group, the process comprising either

(i) reacting an amine of the formula Y-H, where Y is as defined for general formula 11 above with a compound of general formula 8:

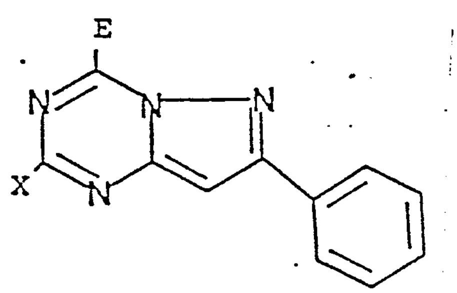

(8)

wherein E represents a leaving group and X represents a thioalkyl group; or

(ii) condensing a compound of general formula 9:

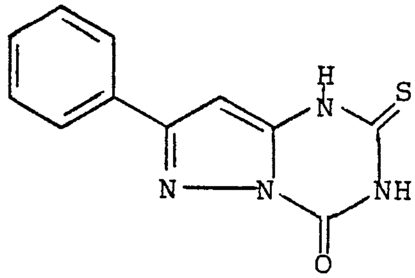

(9)

with a condensing agent which alkylates, aralkylates or thioheteroaralkylates of the sulphur atom and optionally substituting the nitrogen atom which is between and next to the carbon atoms bearing the oxygen and sulphur atoms with a carboalkoxy, alkyl, allyl or benzyl group, or

(iii) converting a compound of formula 11 so formed into a further compound of formula 11;

(iv) and optionally, in any of the above cases, forming a pharmaceutically or veterinarily acceptable salt of the compound.

10. A process for the preparation of a compound of general formula 1:

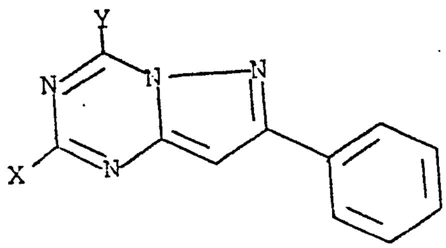

(1)

wherein X represents a thioalkyl group and Y represents a hydroxyalkylamino, dialkylamino, carboxyalkylamino, N-heterocycloalkyl, heteroalkylthioalkylamino, alkylthiocarboxyalkylamino or heteroarylalkylthioalkylamino group;

the process comprising reacting an amine of the formula Y-H, wherein Y is as defined for general formula 1 above with a compound of general formula 8

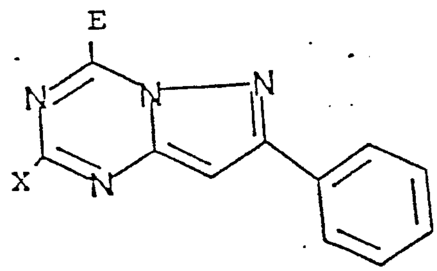

(8)

wherein E represents a leaving group and X is defined for general formula 1 above,

and optionally forming a pharmaceutically or veterinarily acceptable salt of the compound.

11. A process for the preparation of a compound of general formula 2,

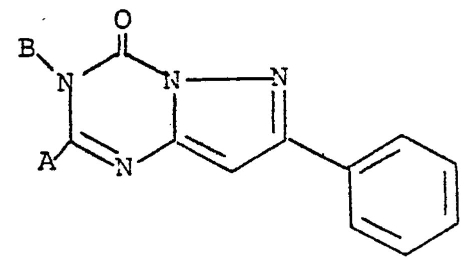

(2)

wherein A represents thioalkyl, thioaralkyl, or thioheteroaralkyl group; and B represents a hydrogen atom or a carboalkoxy, alkyl, allyl or benzyl group; the process comprising condensing a compound of general formula 9

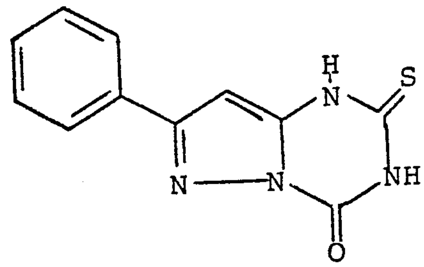

(9)

with a condensing agent which alkylates, aralkylates or thioheteroaralkylates of the sulphur atom and optionally substituting the nitrogen atom which is between and next to the carbon atoms bearing the oxygen and sulphur atoms with a carboalkoxy, alkyl, allyl or benzyl group, and optionally forming a pharmaceutically or veterinarily acceptable salt of the compound.

**Claims for contracting state: AT**

1. A process for the preparation of a compound of general formula 11:

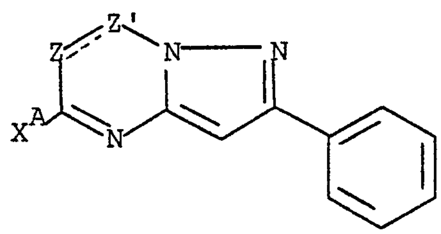

(11)

wherein either $X^A$ represents a thioalkyl group and the

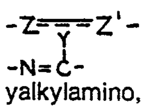 moiety represents , wherein Y represents a hydroxyalkylamino, dialkylamino, carboxyalkylamino, N-heterocycloalkyl, heteroalkylthioalkylamino, alkylthiocarboxyalkylamino or heteroarylalkylthioalkylamino group,

or $X^A$ represents a thioalkyl thioaralkyl or thioheteroaralkyl group and the -Z===Z'- moiety represents 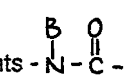, wherein B represents a hydrogen atom or a carboalkoxy, alkyl, allyl or benzyl group, the process comprising either

(i) reacting an amine of the formula Y-H, where Y is as defined for general formula 11 above with a compound of general formula 8:

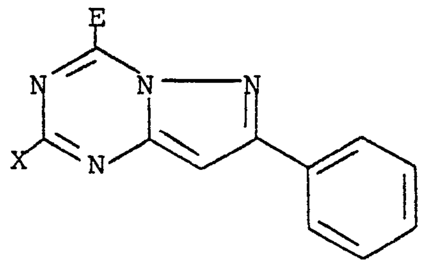

(8)

wherein E represents a leaving group and X represents a thioalkyl group; or

(ii) condensing a compound of general formula 9:

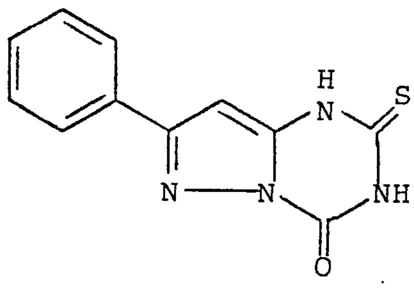

(9)

with a condensing agent which alkylates, aralkylates or thioheteroaralkylates at the sulphur atom and optionally substituting the nitrogen atom whichis between and next to the carbon atoms bearing the oxygen and sulphur atoms with a carboalkoxy, alkyl, allyl or benzyl group, or

(iii) converting a compound of formula 11 so formed into a further compound of formula 11;

(iv) and optionally, in any of the above cases, forming a pharmaceutically or veterinarily acceptable salt of the compound.

2. A process for the preparation of a compound of general formula 1:

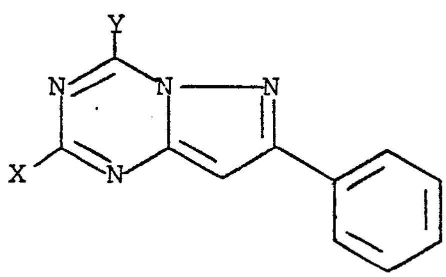

(1)

wherein X represents a thioalkyl group and Y represents a hydroxyalkylamino, dialkylamino, carboxyalkylamino, N-heterocycloalkyl, heteroalkylthioalkylamino, alkylthiocarboxalkylamino or heteroarylalkylthioalkylamino group;

the process comprising reacting an amine of the formula Y-H, wherein Y is as defined for general formula 1 above with a compound of general formula 8

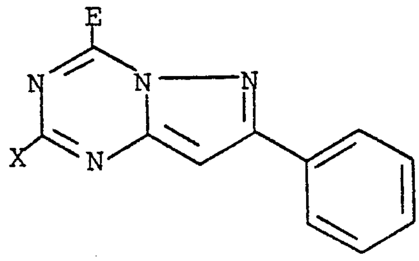

(8)

wherein E represents a leaving group and X is defined for general formula 1 above,

and optionally forming a pharmaceutically or veterinarily acceptable salt of the compound.

3. A process for the preparation of a compound of general formula 2,

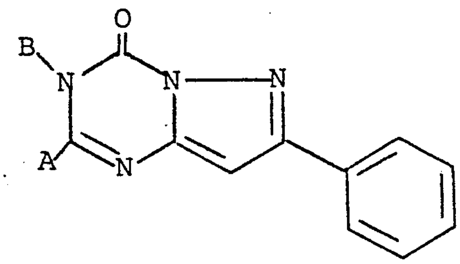

(2)

wherein A represents thioalkyl, thioaralkyl, or thioheteroaralkyl group; and B represents a hydrogen atom or a carboalkoxy, alkyl, allyl or benzyl group;

the process comprising condensing a compound of general formula 9

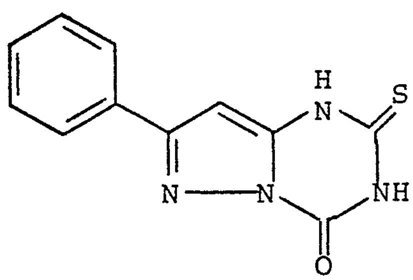

(9)

with a condensing agent which alkylates, aralkylates or thioheteroaralkylates at the sulphur atom and optionally substituting the nitrogen atom which is between and next to the carbon atoms bearing the oxygen and sulphur atoms with a carboalkoxy, alkyl, allyl or benzyl group, and optionally forming a pharmaceutically or veterinarily acceptable salt of the compound.

4. A process for the preparation of 4-[(3-methylthio-1-carboxy)] propylamino-2-methylthio-7-phenyl-pyrazolo-[1,5-a]-1,3,5-triazine which comprises reacting methionine with 4-methoxy-2-methylthio-7-pyrazolo-[1,5-a]-1,3,5-triazine.

5. A process for the preparation of 2-methylthio-7-phenyl-4-[2-[(2-pyridyl)methylthio]-ethyl amino]-pyrazolo-[1,5-a]-1,3,5-triazine which comprises reacting 2-pyridylmethylthioethylamine dihydrobromide with 4-methoxy-2-methylthio-7-phenylpyrazolo-[1,5-a]-1,3,5-triazine.

6. A process for the preparation of 2-(2-pyridylmethyl)thio-7-phenylpyrazolo [1,5-a]-1,3,5-triazine-4-one which comprises reacting 2-picolyl chloride hydrochloride with 7-phenyl-2-thiopyrazolo [1,5-a]-1,3,5-triazine-4-one.

7. A process for the preparation of 3-carbethoxy-methyl-2-2-methylthio-7-phenylpyrazolo-[1,5 -a]-1,3,5-triazine-4-one which comprises reacting ethylbromacetate with 7-phenylpyrazolo-[1,5-a]-1,3,5-triazine-4-one.